Europäisches Patentamt

European Patent Office

Office européen des brevets

(19)

(11) Numéro de publication : **0 325 874 B1**

(12)

# FASCICULE DE BREVET EUROPEEN

(45) Date de publication du fascicule du brevet :
**15.04.92 Bulletin 92/16**

(51) Int. Cl.⁵ : **G01N 33/49,** G01N 11/16

(21) Numéro de dépôt : **88403279.8**

(22) Date de dépôt : **22.12.88**

(54) **Procédé et dispositif pour déterminer le temps de modification de l'état physique d'un milieu fluide.**

(30) Priorité : **30.12.87 FR 8718348**
**06.07.88 FR 8809151**

(43) Date de publication de la demande :
**02.08.89 Bulletin 89/31**

(45) Mention de la délivrance du brevet :
**15.04.92 Bulletin 92/16**

(84) Etats contractants désignés :
**AT BE CH DE ES GB GR IT LI LU NL SE**

(56) Documents cités :
**EP-A- 0 015 550**
**EP-A- 0 169 794**
**DE-A- 2 112 055**
**FR-A- 2 465 227**
**REVIEW OF SCIENTIFIC INSTRUMENTS,**
**vol.55, no.4, avril 1984; C.LEYH et al.,**
**pp.570-577**

(73) Titulaire : **SERBIO**
**30, Avenue Flachat**
**F-92600 Asnières (FR)**

(72) Inventeur : **Martinoli, Jean-Luc**
**2, Rue du Tremble**
**F-92390 Villeneuve la Garenne (FR)**
Inventeur : **Rousseau, Alain**
**7, Rue Nicolas Houel**
**F-75005 Paris (FR)**
Inventeur : **Vilain, Pascal**
**27, Rue Croix du Bellay**
**F-60240 Hadancourt le Haut Clocher (FR)**

(74) Mandataire : **Marquer, Francis et al**
**Cabinet Moutard 35, Avenue Victor Hugo**
**F-78960 Voisins le Bretonneux (FR)**

EP 0 325 874 B1

## Description

La présente invention concerne un procédé pour déterminer le temps de modification de l'état physique d'un milieu fluide, et en particulier pour déterminer le temps de coagulation du sang, du genre dans lequel une bille ferro-magnétique est placée dans le fond d'un godet contenant le fluide à étudier et entraînée dans un mouvement d'oscillation dans un plan vertical sous l'effet d'un champ magnétique extérieur, et dans lequel on détecte les variations de l'amplitude et/ou de la fréquence de mouvements de cette bille dues à la modification de l'état physique. L'invention concerne également un dispositif pour la mise en oeuvre de ce procédé.

Un procédé de mesure de la viscosité de ce genre est décrit notamment dans le document (REVIEW OF SCIENTIFIC INSTRUMENTS (D1).

La bille est maintenue en suspension dans le fluide, puis entraînée en oscillation forcée au moyen d'un champ magnétique. Le dispositif d'asservissement nécessaire et les règlages sont délicats. Enfin, le volume de fluide nécessaire est trop important pour les applications au sang.

Dans le brevet européen N° 90.192, on décrit un dispositif dans lequel la bille se déplace librement sur le fond plat et horizontal du godet. Lorsqu'intervient la modification d'état physique du fluide qui est contenu dans ce dernier, et qui est ici du plasma sanguin additionné de réactifs, la bille se déplace sur une courbe dirigée vers l'intérieur à cause, semble-t-il, de l'accroissement de la résistance mécanique opposée par le contenu du godet, et on détecte son déplacement radial par des moyens bien connus en eux-mêmes.

La précision des mesures ainsi réalisées laisse à désirer, parce que le déplacement radial d'une bille vers le centre d'un godet de très faibles dimensions est une grandeur physique qui est difficile à à détecter et à évaluer de manière satisfaisante. En outre, la reproductibilité de telles mesures n'est pas bonne, à cause notamment de la complexité des phénomènes qui leur servent de base.

Selon EP A 0 169 794 (A. GIROLAMI), la bille est guidée par un chemin de roulement et entraînée mécaniquement dans un mouvement circulaire non rotatif. Son ralentissement est irrégulier et, en pratique, son arrêt - lequel semble imprécis et aléatoire - peut être semble détecté.

Aussi, le but de la présente invention est de proposer un procédé pour mesurer le temps de modification de l'état physique d'un milieu fluide du genre spécifié ci-avant qui pallie les inconvénients des dispositifs connus et, en particulier, qui permette des mesures dont la précision, la fiabilité et la reproductibilité soient supérieures à celles obtenues jusqu'alors avec des appareils de ce type.

D'autre part, un objet de l'invention est un dispositif pour la mise en oeuvre d'un tel procédé qui soit pratiquement automatique et dans lequel l'intervention humaine soit réduite au strict minimum, sans que son coût soit prohibitif pour autant.

Le but ainsi défini, ainsi que d'autres qui apparaîtront par la suite, sont atteints selon l'invention grâce à un procédé du genre dans lequel une bille ferro-magnétique est placée dans un godet contenant le fluide à étudier et entraînée dans un mouvement d'oscillation dans un plan vertical sous l'effet d'un champ magnétique extérieur, et dans lequel on détecte les variations de l'amplitude et/ou de la fréquence des mouvements de cette bille dues à la modification de l'état physique et caractérisé par le fait que la bille est guidée par un chemin de roulement non rectiligne dans un mouvement pendulaire et que la fréquence d'excitation du champ magnétique est voisine de la fréquence naturelle d'oscillation de la bille dans le milieu fluide le long dudit chemin de roulement. Lorsque le fluide à étudier subit une modification de son état physique qui augmente sa viscosité, et, par exemple, lorsqu'il se coagule s'il s'agit de plasma sanguin additionné de réactifs appropriés, la bille est freinée dans son mouvement, puis arrêtée, de sorte que l'amplitude de ses mouvements périodiques diminue brusquement. On détecte cette diminution d'amplitude, et ceci permet de déterminer le moment où la modification a lieu.

On comprend que le fait que la bille suive, à chaque période, un trajet imposé toujours identique permet une grande précision et une bonne reproductibilité des mesures, puisqu'il suffit de détecter les variations d'une période, et donc d'une fréquence électrique, ou d'une amplitude, et donc de la valeur d'un signal électrique, ce qui est une opération aisée pour les spécialistes en la matière. En outre, cette détection est, de toute évidence, plus facile et moins entachée d'erreurs que celle qui consiste à déterminer l'échappement vers le centre du godet d'une bille de très faibles dimensions.

Le mouvement de la bille dans un plan vertical bien déterminé, et non plus, de manière quasi aléatoire, sur le fond plat d'un godet comme dans le brevet européen N° 90 192 précité, augmente encore la précision des mesures.

De préférence, la fréquence et/ou l'intensité du champ magnétique extérieur qui commande ce mouvement oscillatoire est règlable.

Le règlage de l'intensité permet de faire varier la force de l'agitation créée par cette bille dans le fluide à étudier.

De préférence, la détermination du temps de modification de l'état physique évoquée ci-avant est associée à une mesure densitométrique. L'association de ces deux appareils dans une même unité simplifie notablement un certain nombre d'opérations. Elle permet en outre de diminuer les coûts, du fait que l'on peut utiliser à deux fins différentes divers organes annexes, comme les dispositifs de régulation de la température, les alimentations et/ou une partie au moins des interfaces homme/machine, ces organes pouvant en outre être communs à plusieurs godets de mesure logés dans le même appareil.

En outre, cette association d'une mesure de densité optique et d'une détermination du temps de modification de l'état physique prend toute sa valeur si, selon un mode de réalisation particulièrement avantageux de la présente invention, le faisceau lumineux utilisé pour la première de ces mesures rencontre la trajectoire de la bille spécifiée ci-avant et sert à la seconde. En effet, les oscillations de la bille se traduisent par des variations d'éclairement du détecteur densitométrique qui sont aisément utilisés pour déterminer l'amplitude de ces oscillations.

Ainsi, le même faisceau lumineux et le même détecteur, qui est par exemple une photodiode, peuvent être utilisés aussi bien pour déterminer le temps de modification de l'état physique que pour mesurer la densité optique.

En ce qui concerne le dispositif permettant la mise en oeuvre de la présente invention, le fond du godet destiné à contenir la bille ferromagnétique spécifiée ci-avant présente avantageusement un chemin de roulement curviligne concave vers le haut dont le point le plus bas est en son centre.

On comprend qu'ainsi la bille, lorsqu'elle est abandonnée à elle-même depuis le point le plus haut de son chemin de roulement, accomplit sur ce dernier le mouvement oscillatoire de l'invention. Toutefois, ce mouvement s'amortit généralement assez vite à cause de la viscosité des fluides à étudier.

Aussi, le dispositif selon l'invention comprend avantageusement deux pôles magnétiques qui sont placés de part et d'autre du chemin de roulement spécifié ci-avant, à l'extérieur du godet, et dont chacun est entouré par une bobine magnétique, ainsi que des moyens pour envoyer périodiquement et alternativement dans ces dernières un courant électrique de période et/ou d'intensité réglable.

Mis à part sa fonction d'entretien du mouvement de la bille, cette disposition permet de régler la période et la force de ce mouvement, ce qui est avantageux, ainsi que cela a été dit plus haut. On notera en outre que, par rapport au dispositif du brevet européen précité, on supprime ici tout organe tournant, ce qui procure des avantages évidents.

D'autre part, les pôles magnétiques spécifiés ci-avant terminent avantageusement une carcasse magnétique en forme de U dont chaque branche est entourée par une bobine, de sorte que le circuit magnétique qui vient d'être décrit est fermé, ce qui rend minimales les fuites magnétiques.

Selon une forme de réalisation avantageuse du dispositif selon l'invention, ce sont les variations d'amplitude des oscillations de la bille que l'on détecte, et ce, de préférence, au moyen d'un faisceau lumineux qui traverse le godet où la bille se déplace, et dont l'occultation varie en fonction de la position de la bille. Il suffit alors de transformer les signaux optiques correspondant à cette occultation en des signaux électriques, au moyen d'une photodiode par exemple, pour détecter aisément les modifications du mouvement de la bille.

De préférence, le godet selon l'invention présente, aux extrémités de son chemin de roulement, des faces planes, transparentes et sensiblement parallèles. Ceci permet de l'utiliser simultanément pour un densitomètre, le rayon lumineux qui vient frapper le détecteur de ce dernier traversant alors aisément le godet, et ce, sans distorsions ni pertes.

Mais, en ce qui concerne ce point, et selon une forme particulièrement avantageuse de l'invention, le chemin de roulement curviligne prévu pour la bille est placé dans le dispositif de telle manière que la trajectoire de cette dernière recoupe le faisceau lumineux du densitomètre, et le dispositif comprend des moyens pour traduire l'occultation de ce faisceau en un signal électrique. Ici, l'expression "recoupe" veut dire que la trajectoire de la bille n'est ni totalement extérieure, ni totalement intérieure au faisceau lumineux. Il en résulte que les mouvements oscillatoires de la bille se traduisent en des signaux électriques qui peuvent être aisément exploités pour déterminer la période des oscillations de la bille et, de ce fait, pour mesurer le temps de modification de l'état physique de fluide contenu dans le godet.

De préférence, et pour augmenter la précision de la mesure, le chemin de roulement de la bille est disposé dans l'appareil de telle façon que cette dernière, lorsqu'elle se trouve au point le plus bas du chemin de roulement, est sensiblement tangente par son sommet au faisceau lumineux qui éclaire le détecteur du densitomètre. Le taux d'occultation de ce faisceau varie alors entre zéro et une valeur maximale atteinte lorsque la bille se trouve en l'un des points les plus hauts du chemin de roulement, une valeur maximale de 20% étant largement suffisante pour obtenir une excellente précision.

Ainsi que cela a été dit plus haut, le fond du godet destiné à contenir cette bille présente, selon l'invention, un chemin de roulement curviligne concave vers le haut dont le point le plus bas est en son centre, ce qui le distingue des godets de ce genre appartenant à l'art antérieur, comme ceux à fond plat qui sont commercialisés par les sociétés ISABIOLOGIE et BEHRING, et ceux dont le fond comporte une gouttière anulaire et qui sont décrits dans les brevets FR-A-2.465.227 et FR-A-2.566.908.

Avantageusement, ce godet comprend à sa partie supérieure un orifice d'introduction des réactifs qui débouche dans une cuvette sensiblement parallélépipédique très allongée dans le sens du chemin de roulement que présente son fond. En d'autres termes, cette cuvette comporte deux grandes faces très rapprochées l'une de l'autre, la distance entre elles étant à peine supérieure au diamètre de la bille qu'elle doit contenir, et deux petites faces, de part et d'autre du chemin de roulement, éloignées entre elles de plusieurs fois ce diamètre.

Selon une forme de réalisation de l'invention, le chemin de roulement que comporte le fond de cette cuvette peut être une portion de cylindre dans laquelle est ménagée une rainure de guidage de la bille.

Selon une autre forme de réalisation, le fond de cette cuvette est une surface concave à laquelle se raccordent de part et d'autre les deux grandes faces de la cuvette et qui est, de préférence, une surface de révolution dont l'axe est perpendiculaire aux grandes faces en question. La bille est alors ramenée par son propre poids vers la ligne médiane de la gouttière ainsi formée.

Cette surface peut être cylindrique, torique, elliptique ou similaire, ou elle peut présenter en section transversale la forme d'un V ouvert vers le haut.

De préférence, les faces opposées de la cuvette inférieure du godet selon l'invention ne sont pas strictement parallèles entre elles, mais inclinées d'un angle inférieur ou égal à 3 degrés par rapport du plan de symétrie des faces concernées.

L'orifice d'introduction du godet selon l'invention peut être par exemple cylindrique, conique ou parallélépipédique mais, dans ce dernier cas, ses faces latérales présentent entre elles des congés, et non des arêtes vives, afin d'éviter l'accrochage des réactifs par capillarité dans de telles arêtes.

De tels godets selon l'invention font avantageusement partie d'un ensemble ou bloc de plusieurs godets qui sont par exemple moulés d'un seul tenant en une matière plastique appropriée.

La description qui va suivre, et quine présente aucun caractère limitatif, permettra de bien comprendre comment la présente invention doit être mise en pratique. Elle doit être lue en regard des dessins annexés, parmi lesquels:
  – La figure 1 représente une vue en couple verticale schématique d'un dispositif pour la mise en oeuvre du procédé selon la présente invention ;
  – La figure 2 montre une vue en coupe prise selon la ligne II-II de l'objet de la figure 1 ;
  – La figure 3 est une vue de dessous d'un godet suivant l'invention faisant partie d'un ensemble de plusieurs godets ;
  – La figure 4 est une coupe suivant II-II de l'objet de la figure 3 ;
  – La figure 5 est une coupe suivant III-III de l'objet de la figure 3 ;
  – La figure 6 est une vue de dessus d'un ensemble de godets ;
  – La figure 7 est une vue de côté de l'ensemble de godets, objet de la figure 6 ; et :
  – La figure 8 est une vue en perspective d'un godet isolé suivant l'invention.

Dans ce qui va suivre, on décrira l'appareil selon l'invention et son mode de fonctionnement dans le cas où il s'agit de déterminer le temps de coagulation d'un plasma sanguin additionné des réactifs usuels. Toutefois, il est bien claire que cet appareil peut être utilisé à d'autres fins, moyennant, le cas échéant, diverses modifications qui sont à la portée des spécialistes en la matière.

Le coagulomètre représenté sur les figures comprend un godet transparent 1 de forme générale parallélépipédique qui peut être réalisé en une matière plastique adéquate, qui renferme une bille ferromagnétique 2 et qui est destiné à recevoir le plasma à étudier additionné des réactifs habituels. Plus précisément, le godet 1 comporte une cavité plate 3 qui est allongée dans le sens transversal du dispositif et dont le fond présente un chemin de roulement 4 pour la bille 2.

Le chemin de roulement 4 s'étend dans le plan de symétrie vertical du godet 1 dans le sens transversal de ce dernier ; soin profil est un cercle de rayon R qui est centré sur l'axe de symétrie vertical du godet 1 de sorte que son point le plus bas est situé sur cet axe. Il en résulte que la bille 2, lorsqu'elle est abandonnée à elle-même depuis le point le plus haut de chemin de roulement 4 accomplit sur ce dernier un mouvement pendulaire de période $T_0$ qui dépend du rayon de courbure R du chemin de roulement 4. Enfin, le diamètre de la bille 2 est légèrement inférieur à la largeur de la cavité 3 et le chemin de roulement 4 est constitué par une rainure 5 ménagée dans le fond du godet 1.

De part et d'autre du chemin de roulement 4 sont placés à l'extérieur du godet 1 deux pôles magnétiques 6a et 6b qui terminent une carcasse magnétique 7 en forme de U dont chaque branche est entourée par une bobine 8a, 8b.

Le densitomètre optique qui est associé à ce coagulomètre n'a pas été représenté de manière détaillée ; il comprend, de part et d'autre du godet 1, une diode émettrice électroluminescente 9 et une photodiode réceptrice 10 devant laquelle est placé un filtre optique passe-bande 11. Le faisceau lumineux correspondant 12 s'étend de manière à être sensiblement tangent à la bille 2 lorsque cette dernière se trouve au point le plus bas de son chemin de roulement 4.

Selon une forme de réalisation particulière donnée à titre non limitatif, la diode électroluminescente 9, dont l'intensité lumineuse maximale se situe vers 480 nm, est alimentée à partir d'un microprocesseur par un courant régulé et haché à quelques kilohertz, et ce, à travers le filtre 11 qui transmet la lumière jusqu'à 500 nm environ. Cette diode 9 éclaire la photodiode 10 avec laquelle elle est placée en ligne, de façon que le faisceau lumineux traverse le plasma et le godet 1. Les informations fournies par la photodiode 10 sont transmises au microprocesseur précité à travers un filtre qui permet de ne retenir que le signal haché émis par la source 9.

D'autre part, ainsi que cela a été dit plus haut, le godet 1 est de préférence disposé dans l'appareil de telle manière que le faisceau lumineux 12 du densitomètre soit sensiblement tangent à la bille 2 lorsqu'elle se trouve au point le plus bas du chemin de roulement 4.

Le mode de fonctionnement de ce dispositif est le suivant. On commence par verser dans le godet 1 le plasma à étudier additionné des réactifs usuels, et ce, jusqu'à un niveau tel qu'il recoupe le faisceau lumineux 12.

On envoie par ailleurs alternativement dans les bobines 8a et 8b un courant tel que la bille 2 accomplisse sur son chemin de roulement 4 un mouvement pendulaire de période T voisine de sa période propre d'oscillation. Lorsque le sang se coagule, l'amplitude de ses oscillations diminue brutalement et la détermination du moment où ce phénomène se produit permet celle du temps de coagulation.

Quant à la détermination du moment où le mouvement périodique de la bille 2 s'arrête, on se rappelle que le faisceau lumineux 12 du densitomètre est sensiblement tangent à cette bille lorsqu'elle se trouve au point le plus bas de son chemin de roulement 4. Il en résulte qu'elle occulte partiellement le faisceau 12 lorsqu'elle s'en écarte, l'occultation maximale ayant lieu bien entendu lorsque la bille 2 atteint son point le plus haut où elle est représentée en traits interrompus en 2'. Il suffit que 20% de la surface du faisceau 12 soit occultée lorsque la bille 2 est en son point le plus haut pour que l'on obtienne une excellente précision pour les mesures.

On comprend qu'il suffit d'interposer à la sortie de la photodiode réceptrice 10 un circuit électronique approprié pour détecter l'arrêt de la bille 2 lors de la coagulation du plasma contenu dans le godet 1. Un tel circuit est à la portée des spécialistes en la matière, et il ne sera pas décrit ici de manière détaillée.

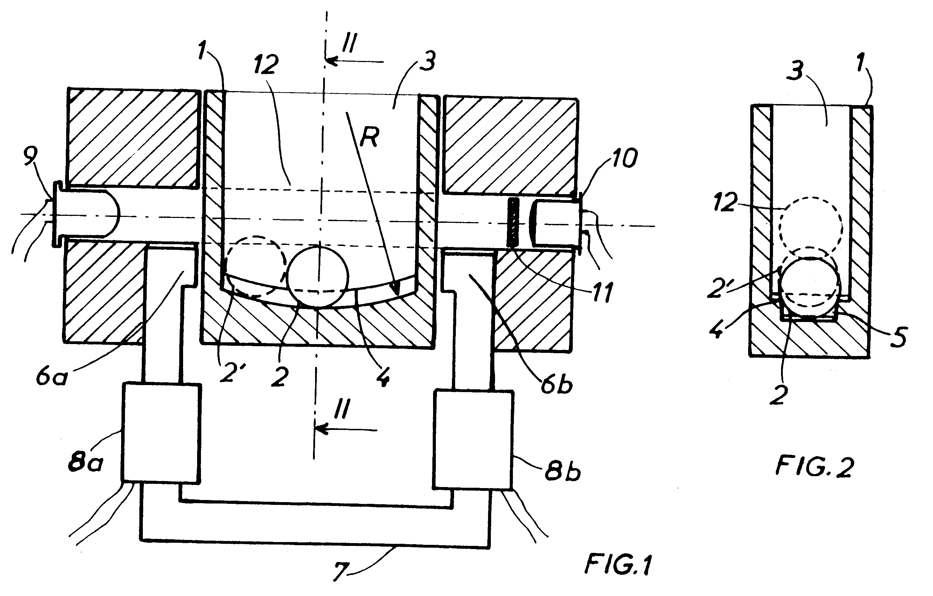

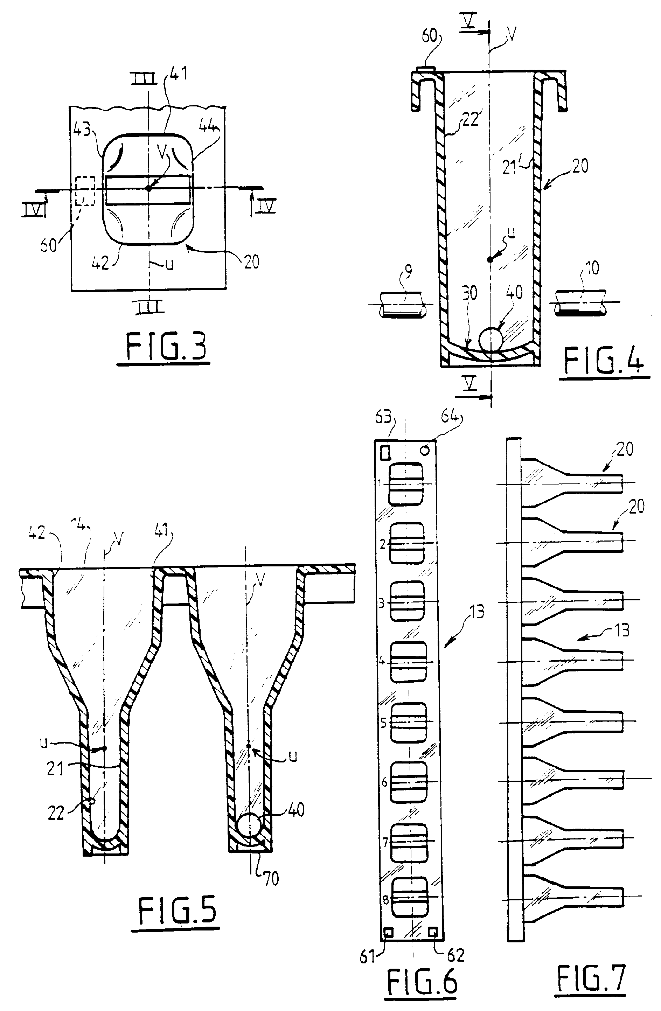

On décrira maintenant, en regard des figures 3 à 8, des variantes du godet qui est représenté sur les figures 1 et 2.

Chaque godet 20 des figures 3 à 7 fait partie d'un ensemble ou bloc 13 du plusieurs godets. La godet 20 présente à sa partie inférieure une cuvette comportant deux parois latérales rapprochées 21 et 22 opposées délimitant avec le fond 30 un chemin de roulement pour la circulation d'une bille 40, d'une part, et deux autres parois latérales opposées 21' et 22' séparées l'une de l'autre par une distance plus grande que celle séparant les parois rapprochées 21, 22, d'autre part.

Les parois 21 et 22 sont sensiblement parallèles entre elles. Plus précisément, elles sont soit parallèles, soit légèrement inclinées par rapport à l'axe vertical de symétrie V (ou plan de symétrie II) de la cuvette. De même, les parois 21' et 22' sont sensiblement parallèles entre elles, c'est-à-dire qu'elles sont soit parallèles, soit également légèrement inclinées par rapport audit axe de symétrie V. De façon avantageuse, on préfère que les parois 21 et 22, d'une part, et 21' et 22', d'autre part, soient légèrement inclinées par rapport à l'axe V de façon que l'espace entre ces parois se rétrécisse du haut vers le bas de la cuvette. L'angle de l'inclinaison de ces parois par rapport à l'axe vertical de symétrie V sera avantageusement inférieur ou égal à 3 degrés, et mieux inférieur ou égal à 1 degré.

Dans le cas où la surface concave du fond 30 est de révolution, son axe de révolution U est perpendiculaire au plan vertical de symétrie II de la cuvette. Cette surface concave est avantageusement, comme indiqué ci-dessus, soit cylindrique, soit torique.

Le godet 20 présente à sa partie supérieure un dispositif 14 d'introduction de réactif par pipettage ayant la forme d'un entonnoir. De façon avantageuse, on préfère que ce dispositif 14 présente au niveau de son ouverture des parois opposées 41 et 42, d'une part, et 43 et 44, d'autre part, qui sont légèrement inclinées par rapport à l'axe V.

On préfère également que la liaison de chaque couple de parois 41/42, 41/44, 42/43 et 42/44 ne soit pas constituée par une arête trop vive mais que cette liaison soit curviligne. En pratique, on recommande que le godet comporporte à sa partie supérieure un orifice d'introduction 14 délimité par des faces opposées 41 et 42, d'une part, et des faces opposées 43 et 44, d'autre part, qui soit sensiblement carré ou rectangulaire. Un tel agencement facilite l'introduction d'un ou plusieurs réactifs par pipettage dès lors que la présence d'arêtes vives empêcherait par des phénomènes de tension superficille l'écoulement du ou des réactifs vers la partie inférieure du godet. Avec plusieurs liaisons curvilignes, on peut repérer facilement la localisation des différents réactifs à introduire.

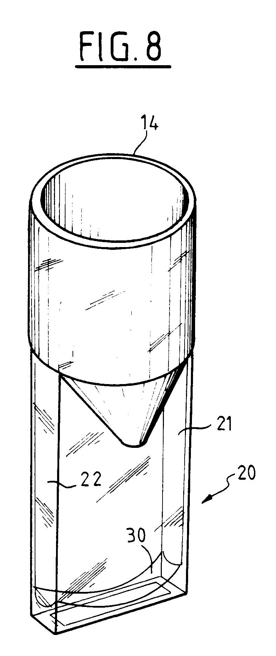

En variante, comme représenté à la figure 8, le dispositif d'introduction 14 peut être cylindrique ou tronconique.

L'ensemble 13 de godets peut comporter au moins un moyen de repérage 60 (figures 3 et 4). Ce moyen de repérage peut être par exemple, suivant la figure 6, une indication calendaire telle que l'année, 61, une indication calendaire telle que le mois dans l'année, 62, un sigle ou monogramme en relief 63 du fabricant du bloc de godets, ou encore un moyen de marquage distinctif 64 de ce bloc.

Par ailleurs, le bloc 13 de godets peut comporter des indications, par exemple des numéros ou des lettres, représentées par les chiffres 1 à 8 de la figure 6, pour distinguer chacun des godets du bloc.

Chaque godet 20 ou chaque bloc 13 de godets peut être conçu en un matériau approprié, notamment en verre ou, mieux, en matière plastique. De préférence chaque godet ou chaque bloc de godets est réalisé en matière plastique, notamment selon une technique de moulage par injection au niveau du repère 70 de la figure 5.

Les blocs ou ensembles 13 de godets 20 selon l'invention peuvent être utilisés dans les analyseurs automatiques et semi-automatiques. Quand on utilise un analyseur automatique capable d'effectuer une série de mesures différentes, on préfère utiliser des cuvettes 10 isolées, dites unitaires, l'analyseur automatique pouvant ainsi effectuer des mesures rapides sur des échantillons différents en fonction des urgences. D'une manière pratique, les analyseurs automatiques du commerce fonctionnent généralement avec deux pipettages, c'est-à-dire qu'ils permettent l'introduction de deux réactifs différents ; l'absence d'arêtes vives à la partie supérieure des godets 20 selon l'invention, c'es-à-dire au niveau de leur dispositif d'introduction 14, permet efficacement les deux pipettages en question.

Sans sortir du cadre de la présente invention, il serait possible d'apporter diverses modifications au mode de réalisation qui vient d'être décrit. C'est ainsi, par exemple, que l'on pourrait utiliser un capteur inductif, capacitif ou opto-électronique au lieu de faire appel au détecteur du densitomètre.

## Revendications

1. Procédé pour déterminer le temps de modification de l'état physique d'un milieu fluide, et en particulier pour déterminer le temps de coagulation du sang, du genre dans lequel une bille ferromagnétique est placée dans le fond d'un godet contenant le fluide à étudier et entraînée dans un mouvement d'oscillation dans un plan vertical sous l'effet d'un champ magnétique extérieur, et dans lequel on détecte les variations de l'amplitude et/ou de la fréquence des mouvements de cette bille dues à la modification de l'état physique, caractérisé par le fait que la bille est guidée par un chemin de roulement non rectiligne dans un mouvement pendulaire et que la fréquence d'excitation du champ magnétique est voisine de la fréquence propre d'oscillation de la bille dans le milieu fluide lelong dudit chemin de roulement.

2. Procédé selon la revendication 1, caractérisé par le fait que la période et/ou l'intensité dudit champ magnétique extérieur est règlable.

3. Procédé selon l'une quelconque des revendications 1 à 2, caractérisé par le fait que la détermination du temps de modification de l'état physique est associée à une mesure densitométrique.

4. Procédé selon la revendication 3, caractérisé par le fait que le faisceau lumineux utilisé pour ladite mesure densitométrique rencontre la trajectoire de ladite bille et sert à la détermination du temps de modification de l'état physique.

5. Dispositif pour la mise en oeuvre du procédé selon l'une quelconque des revendications 1 à 4, caractérisé par le fait que le fond dudit godet (1) présente un chemin de roulement curviligne (4) et concave vers le haut pour ladite bille (2), chemin dont le point le plus bas est en son centre.

6. Dispositif selon la revendication 5, caractérisé par le fait que ledit godet (1) comprend à sa partie supérieure un orifice (14) pour l'introduction des réactifs qui débouche dans une cuvette sensiblement parallèlépipédique allongée dans le sens du chemin de roulement (4) que présente le fond.

7. Dispositif selon l'une quelconque des revendications 5 et 6, caractérisé par le fait que ledit chemin de roulement (4) est une portion de cylindre dans laquelle est ménagée une rainure (5) de guidage de la bille (2).

8. Dispositif selon l'une quelconque des revendications 5 et 6, caractérisé par le fait que le fond (30) de ladite cuvette est une surface de révolution dont l'axe est perpendiculaire aux grandes faces (21, 22) de ladite cuvette.

9. Dispositif selon la revendication 5, caractérisé par le fait que ladite surface du fond (30) est choisie parmi l'ensemble comprenant les surfaces cylindriques, toriques ou similaires, et les surfaces ayant une section transversale en forme de V ouvert vers le haut.

10. Dispositif selon l'une quelconque des revendications 5 à 9, caractérisé par le fait que les faces opposées (21, 22 ; 21′, 22′) de la cuvette inférieure dudit godet (1, 20) sont inclinées d'un angle au plus égal à 3° par rapport au plan de symétrie des faces concernées.

11. Dispositif selon l'une quelconque des revendications 5 à 10, caractérisé par le fait que ledit orifice d'introduction (14) est parallélépipédique et que les faces intérieures de ses parois latérales sont reliées entre elles par des congés.

12. Dispositif selon l'une quelconque des revendications 5 à 11, caractérisé par le fait que ledit godet (20) fait partie d'un bloc (13) de plusieurs godets d'un seul tenant.

13. Dispositif selon l'une quelconque des revendications 5 à 12, caractérisé par le fait qu'il comprend deux pôles magnétiques (6a, 6b) qui sont placés de part et d'autre dudit chemin de roulement (4), à l'extérieur dudit godet (1), et dont chacun est entouré par une bobine magnétique (8a, 8b), ainsi que des moyens pour envoyer périodiquement et alternativement dans ces dernières un courant électrique de période et/ou d'intensité règlable.

14. Dispositif selon la revendication 13, caractérisé par le fait que lesdits pôles magnétiques (6a, 6b) terminent une carcasse magnétique (7) en forme de U dont chaque branche est entourée par l'une desdites bobines (8a, 8b).

15. Dispositif selon l'une quelconque des revendications 13 et 14,
caractérisé par le fait qu'il comprend des moyens pour détecter les variations d'amplitude des oscillations de ladite bille (2).

16. Dispositif selon la revendication 15,
caractérisé par le fait que lesdits moyens pour détecter les variations d'amplitude des oscillations de ladite bille (2) comprennent un faisceau lumineux (12) dont l'occultation varie en fonction de la position de la bille (2), des moyens pour transformer cette occultation en signaux électriques, et des moyens pour exploiter ces derniers.

17. Dispositif selon la revendication 16,
caractérisé par le fait que ledit godet (1) présente, aux extrémités de son chemin de roulement (4), des faces planes, transparentes et sensiblement parallèles entre elles qui sont traversées par ledit faisceau lumineux (12).

18. Dispositif selon l'une quelconque des revendications 13 à 17,
caractérisé par le fait qu'il comporte en outre un densitomètre.

19. Dispositif selon la revendication 18,
caractérisé par le fait que ledit chemin de roulement (4) est placé de telle manière que la trajectoire de ladite bille (2) recoupe le faisceau lumineux dudit densitomètre, et qu'il comprend des moyens pour traduire l'occultation de ce faisceau en un signal électrique.

20. Dispositif selon l'une des revendications 18 et 19,
caractérisé par le fait que ledit godet (1) est disposé de telle façon que ladite bille (2), lorsqu'elle se trouve au point le plus bas de son chemin de roulement (4), est sensiblement tangente par son sommet au faisceau lumineux qui éclaire le détecteur (10) du densitomètre.

**Patentansprüche**

1. Verfahren zum Bestimmen der Zeitspanne, die eine Flüssigkeit zur Änderung ihres physischen Zustandes benötigt, insbesondere zur Bestimmung der Gerinnungszeit von Blut, des Typs, in dem eine ferromagnetische Kugel auf dem Boden eines Bechers angeordnet ist, welcher die zu untersuchende Flüssigkeit enthält, und unter dem Einfluss eines äusseren Magnetfeldes in eine oszillierende Bewegung in einer senkrechten Ebene versetzt wird und in dem die durch die Änderung des physischen Zustandes hervorgerufenen Amplituden- und/oder Bewegungsfrequenzschwankungen dieser Kugel erfasst werden,
dadurch gekennzeichnet, dass die Kugel auf einer nicht gradlinigen Rollbahn mit einer Pendelbewegung geführt wird und die Erregungsfrequenz des Magnetfeldes der Eigenoszillationsfrequenz der Kugel in der Flüssigkeit entlang der Rollbahn angenähert ist.

2. Verfahren nach Anspruch 1,
dadurch gekennzeichnet, dass die Periode und/oder die Intensität des besagten äusseren Magnetfeldes einstellbar ist.

3. Verfahren nach einem der Ansprüche 1 bis 2,
dadurch gekennzeichnet, dass die Bestimmung der Änderungzeit des physischen Zustandes einer densitometrischen Messung zugeordnet ist.

4. Verfahren nach Anspruch 3,
dadurch gekennzeichnet, dass das für besagte densitometrische Messung verwendete Lichtbündel auf die Bahn der Kugel trifft und zur Bestimmung der Änderungzeit des physischen Zustandes dient.

5. Vorrichtung zur Anwendung des Verfahrens nach einem der Ansprüche 1 bis 4,
dadurch gekennzeichnet, dass der Boden des besagten Bechers (1) eine gekrümmte, nach oben konkave Rollbahn (4) für die besagte Kugel (2) aufweist, Rollbahn, deren Tiefpunkt in ihrem Zentrum liegt.

6. Vorrichtung nach Anspruch 5,
dadurch gekennzeichnet, dass besagter Becher (1) in seinem oberen Abschnitt eine Öffnung (14) zur Einführung der Reagenzien aufweist, welche in ein, im wesentlichen quaderförmiges, in Richtung der auf dem Boden angeordneten Rollbahn (4) langgestrecktes Gefäss mündet.

7. Vorrichtung nach einem der Ansprüche 5 und 6,
dadurch gekennzeichnet, dass besagte Rollbahn (4) ein Zylinderabschnitt ist, in dem eine Führungsrinne für die Kugel (2) vorgesehen ist.

8. Vorrichtung nach einem der Ansprüche 5 und 6,
dadurch gekennzeichnet, dass der Boden (30) des Gefässes eine Rotationsfläche ist, deren Achse senkrecht zu den Breitseiten (21, 22) des besagten Gefässes verläuft.

9. Vorrichtung nach Anspruch 5,
dadurch gekennzeichnet, dass besagte Bodenfläche (30) unter verschiedenen zylindrischen, torischen oder ähnlichen Flächen ausgewählt wird und Flächen, welche im Querschnitt die Form eines nach oben hin offenen Vs haben.

10. Vorrichtung nach einem der Ansprüche 5 bis 9,
dadurch gekennzeichnet, dass die gegenüberliegenden Flächen (21, 22 ; 21', 22') des unteren Behälters des besagten Bechers (1, 20) mit einem Winkel von höchstens 3° schräg zur Symmetrieebene der betreffenden Flächen verlaufen.

11. Vorrichtung nach einem der Ansprüche 5 bis 10,
dadurch gekennzeichnet, dass besagte Einführöffnung (14) quaderförmig ausgebildet ist und die Innenflächen ihrer Seitenwände miteinander durch Rundungen verbunden sind.

12. Vorrichtung nach einem der Ansprüche 5 bis 11,
dadurch gekennzeichnet, dass besagter Becher (20) zu einer Einheit (13) aus mehreren zusammenhängenden Bechern gehört.

13. Vorrichtung nach einem der Ansprüche 5 bis 12,
dadurch gekennzeichnet, dass sie zwei Magnetpole (6a, 6b) aufweist, die jeweils auf einer Seite der besagten Rollbahn (4) ausserhalb des Bechers (1) angeordnet sind, und jeder von einer Magnetspule (8a, 8b) umgeben ist, sowie Mittel, um periodisch und abwechselnd in diese Spulen einen elektrischen Strom mit einstellbarer Periode und/oder Stärke einzuspeisen.

14. Vorrichtung nach Anspruch 13,
dadurch gekennzeichnet, dass besagte Magnetpole (6a, 6b) die Enden eines U-förmigen Magnetgehäuses bilden, und jeder Schenkel des U von einer der besagten Spulen (8a, 8b) umgeben ist.

15. Vorrichtung nach einem der Ansprüche 13 und 14,
dadurch gekennzeichnet, dass sie Mittel zur Erfassung von Änderungen der Oszillationsamplitude der besagten Kugel (2) aufweist.

16. Vorrichtung nach Anspruch 15,
dadurch gekennzeichnet, dass zu besagten Mitteln, um Änderungen der Oszillationsamplitude der besagten Kugel (2) festzustellen ein Lichtbündel (12) gehört, dessen Verdeckung sich je nach der Position der Kugel (2) ändert, Mittel, um besagte Verdeckung in elektrische Signale umzusetzen und Mittel, um diese Signale auszuwerten.

17. Vorrichtung nach Anspruch 16,
dadurch gekennzeichnet, dass besagter Becher (1) an den Enden seiner Rollbahn (4) ebene, durchsichtige und im wesentlichen zueinander parallele Flächen aufweist, welche besagtes Lichtbündel (12) durchlassen.

18. Vorrichtung nach einem der Ansprüche 13 bis 17,
dadurch gekennzeichnet, dass sie ausserdem einen Densitometer aufweist.

19. Vorrichtung nach Anspruch 18,
dadurch gekennzeichnet, dass besagte Rollbahn (4) so angeordnet ist, dass die Bahn der besagten Kugel (2) das Lichtbündel des besagten Densitometers schneidet und dass sie Mittel aufweist, die Verdeckung dieses Lichtbündels in ein elektrisches Signal umzusetzen.

20. Vorrichtung nach einem der Ansprüche 18 und 19,
dadurch gekennzeichnet, dass besagter Becher (1) so angeordnet ist, dass der Scheitelpunkt der besagten Kugel (2), wenn diese sich auf dem Tiefpunkt ihrer Rollbahn (4) befindet, im wesentlichen mit dem Lichtbündel, welches den Detektor (10) des Densitometers beleuchtet, tangierend ist.

## Claims

1. Method for determining the modification time of the physical state of a fluid medium, and in particular for determining the coagulation time of blood, of the kind in which a ferromagnetic ball is placed in the bottom of a cup containing the fluid to be studied and driven with an oscillatory movement in a vertical plane under the effect of an external magnetic field, and in which the variations of the amplitude and/or of the frequency of the movements of this ball, which are due to the modification of the physical state, are detected, characterized in that the ball is guided along a non rectilinear travel path for executing a pendular movement and in that the frequency of excitation of the magnetic field is close to the natural oscillation frequency of the ball in the fluid medium along said travel path.

2. Method according to claim 1,
characterized in that the period and/or the intensity of said external magnetic field is adjustable.

3. Method according to anyone of claims 1 and 2,
characterized in that determination of the modification time of the physical state is associated with a densitometric measurement.

4. Method according to claim 3,
characterized in that the light beam used for said densitometric measurement meets the path of said ball and serves for determining the modification time of the physical state.

5. Device for implementing the method according to anyone of the claims 1 to 4,
characterized in that the bottom of said cup (1) has an upwardly concave curvilinear travel path (4) for said ball (2), the lowest point of said path being at its center.

6. The device according to claim 5,
characterized in that said cup (1) has at its upper part an orifice (14) for the introduction of reagents which opens into a substantially parallelepipedic bowl elongate in the direction of the travel path (4) provided by its bottom.

7. The device according to anyone of claims 5 and 6,
characterized in that said travel path (4) is a cylinder portion in which is formed a groove (5) for guiding the ball (2).

8. The device according to anyone of claims 5 and 6,
characterized in that the bottom (30) of said bowl is a surface of revolution whose axis is perpendicular to the large faces (21, 22) of said bowl.

9. The device according to claim 5,
characterized in that said surface of the bottom (30) is chosen from cylindrical, toric or similar surfaces, and surfaces having an upwardly open V cross section.

10. The device according to anyone of claims 5 to 9, characterized in that the opposite faces (21, 22 ; 21', 22') of the lower bowl of said cup (1, 20) are slanted through an angle at most equal to 3° with respect to the plane of symmetry of the faces concerned.

11. The device according to anyone of claims 5 to 10, characterized in that said introduction orifice (14) is parrallelepipedic and the inner faces of its side walls are connected together by beads.

12. The device according to anyone of claims 5 to 11, characterized in that said cup (20) forms part of a block (13) of several cups in a single piece.

13. The device according to anyone of claims 5 to 12, characterized in that it comprises two masnetic poles (6a, 6b) which are placed on each side of said travel path (4), outside said cup (1), and each of which is surrounded by a magnetic coil (8a, 8b) as well as means for periodically and alternately feeding therein an electric current of adjustable period and/or intensity.

14. The device according to claim 13, characterized in that said magnetic poles (6a, 6b) terminate a magnetic U shaped carcass (7), each leg of which is surrounded by one of said coils (8a, 8b).

15. The device according to anyone of claims 13 and 14, characterized in that it comprises means for detecting the amplitude variations of the oscillations of said ball (2).

16. The device according to claim 15, characterized in that said means for detecting the amplitude variations of the oscillations of said ball (2) comprise a light beam (12) whose occultation varies as a function of the position of the ball (2), means for transforming this occultation into electric signals and means for using the latter.

17. The device according to claim 16, characterized in that said cup (1) has, at the ends of its travel path (4), flat and transparent faces substantially parallel to each other through which said light beam (12) passes.

18. The device according to anyone of claims 13 to 17, characterized in that it further comprises a densitometer.

19. The device according to claim 18, characterized in that said travel path (4) is placed so that the path of said ball (2) cuts the light beam of said densitometer and it comprises means for translating the occultation of this beam into an electric signal.

20. The device according to one of claims 18 and 19, characterized in that said cup (1) is disposed so that said ball (2), when it is at the lowest point of its travel path (4), is substantially tangential by its top to the light beam which illuminates the detector (10) of the densitometer.

FIG.1

FIG.2

EP 0 325 874 B1

FIG.3

FIG.4

FIG.5

FIG.6

FIG.7

# FIG. 8